# EUROPEAN PATENT SPECIFICATION

(11) **EP 3 829 449 B1**
(45) Date of publication and mention of the grant of the patent: **28.02.2024**
(21) Application number: 18928551.3
(22) Date of filing: 01.08.2018
(51) Int. Cl.: A61B 10/02, A61B 17/3205, A61B 17/3209

(54) **BIOPSY PUNCH**
BIOPSIESTANZE
POINÇON DE BIOPSIE

(43) Date of publication of application: 09.06.2021
(73) Proprietor: R H L Down Pty Ltd, Airlie Beach, Queensland 4802 (AU)
(72) Inventor: DOWN, Roger, Airlie Beach, Queensland 4802 (AU)
(74) Representative: Brevalex
(86) International application number: PCT/AU2018/050801
(87) International publication number: WO 2020/023992

(56) References cited:
- WO-A1-2005/112777
- WO-A1-2013/166443
- WO-A2-2005/027758
- WO-A2-2005/027758
- WO-A2-2006/121619
- DE-U1-202009 006 159
- IT-A1- AR20 120 014
- KR-A- 20180 058 448
- US-A- 5 123 907
- US-A- 5 922 000
- US-A- 5 961 529
- US-A1- 2008 215 079

## Description

### Technical area

This disclosure relates generally to therapeutic punches for removing a tissue specimen such as from skin.

### Background

In removing a potential cancerous mole or skin cancer (lesion), is it necessary to cut the skin with a specific margin of perceived normal skin around the lesion (be it, benign or malignant), leaving the extracted specimen intact for pathology examination. This is currently performed either with a scalpel or disposable circular or elliptical punches with a maximum diameter of 10mm.

A punch provides a much more accurate extraction than a scalpel, preserving the lesion and ensuring there is the required margin outside the lesion of normal skin. This ensures a complete excision biopsy. Punches also ensure the formation of good vertical edge cuts. Punches are also preferred by General Practitioners especially for small lesions where the use of a scalpel is very difficult. Punches are quick to use and require little skill when compared to a scalpel, where there is considerable risk of accidental over cuts and/or needlessly long incisions.

However, punches have significant disadvantages for excision sizes in excess of 3 mm in diameter. The hole created by the punch is circular. A circular hole was never meant to be closed as a straight line. Attempts to do so always result in ugly puckering, ripples of the surrounding skin or a large dimple of skin at each end of the wound due to "bulking" of tissue at either end of the wound. The magnitude of this deformity increases with increasing punch diameter, even when a distraction technique is used parallel to Langer's lines i.e. the lines of natural elasticity in the skin.

When a circular excision is sutured together, the skin mating surfaces skew and do not bind/heal correctly when sutured. This adversely impacts healing of the skin and produces an adverse cosmetic result. It may also provide pits and entry sites for infection. Further, the intrinsic centrifugal elastic forces created by circular wounds made by a biopsy punch try to restore its natural state. This results in delayed separation of the wound edges when the sutures are removed, and a wide, ugly, often hypertrophic scar; as it has to heal by secondary intention. Where a skin lesion or mole is removed in a visible area such as the face, chest, shoulder, arms or legs, the resulting pucker, dimple, nodule or hypertrophic scar is disfiguring and provides an often permanent, visible cosmetic defect or imperfection, which may never mould out with time. DE 20 2009 006159 U1 discloses a biopsy punch according to the preamble of claim 1.

It is to be understood that, if any prior art publication is referred to herein, such reference does not constitute an admission that the publication forms a part of the common general knowledge in the art, in Australia or any other country.

### Summary

Disclosed herein is a therapeutic punch that may be ideal for all excision biopsy and therapeutic removal of lesions.

The invention relates to a therapeutic punch as defined in claim 1.

The term "therapeutic punch" is to be interpreted broadly throughout this disclosure to mean a punch capable of taking a tissue sample. For example, the therapeutic punch can be used for excision biopsy and/or therapeutic removal of lesions for cosmetic procedures, such as removing an unsightly area of skin such as a mole.

Preventing puckering and dimpling may help to avoid the formation of scar tissue and other disfiguring features.

Disclosed herein is a therapeutic punch comprising: a first cutting region having a first diameter; a second cutting region connected to the first cutting region, the second cutting region having a second diameter that is smaller than the first diameter.

A ratio of the first diameter to the second diameter may range from about 1 :0.25 to about 1 :0.45, such as approximately 1 :0.375. In some embodiments the second diameter may be 5mm or less.

Having the diameter of the first cutting region being larger than the diameter of the second cutting region may allow the second cutting region to remove the "bulked" tissue at either end of the wound where puckering or dimpling would normally reside together with the lesion for histological examination. Further the outer part of a circle at the wound extremity may redistribute the skin elastic forces to allow a flat wound without puckering, dog ears, or dimpling. Experimentation revealed this may help to aid fine line healing, prevents spreading and delayed separation of a wound. In some embodiments disfigurement may be all but eliminated and a great cosmetic result may be achieved.

The term "cutting region" is to be interpreted broadly to mean a region on a cutting structure or element, such as a blade, capable of cutting a section of tissue. The cutting region is not limited to any type of material insofar that the material merely needs to be capable of acting as a cutting region. In this way, the first cutting region can be referred to as a first cutting element and the second cutting region can be referred to as a second cutting element. The first and second cutting regions may be present on the same structure i.e. the same cutting element and may be unitary with one another, or the first and second cutting regions may be provided by separate structures.

The first and second cutting regions may be defined by respective structures, the structures being fixed relative to one another by welding, adhesive, embedding and/or fasteners. Alternatively, the first and second cutting regions may be formed from a single structure. The single structure may extend as a continuous and unbroken wall to form the first and second cutting regions. The second cutting region is formed as a lobe extending from the first cutting region so that an internal volume defined by the second cutting region extends into an internal volume defined by the first cutting region.

Alternatively, in an unclaimed example, the single region may comprise interconnecting slots that allow the single region to interlock with itself to form the first and second cutting regions. The interlock may delineate the internal volume of the first cutting region from the second cutting region.

An embodiment may further comprise wall sections that connect the first cutting region to the second cutting region. The walls may extend from the first cutting region and connect to the second cutting region at a point where imaginary lines extending along the straight walls intersect at a point between a centre of second cutting region and the wall forming the second cutting region. The wall sections may be straight or curved, and/or may include regions that are straight and/or curved i.e. the wall may include a straight section and a curved section. The walls may extend tangentially from the first cutting region. In these embodiments, the first cutting region may have a diameter ranging from about 3 mm to about 8 mm and the second cutting region may have a diameter that ranges from about 25 to about 45%, such as about 37.5%, of the diameter of the first cutting region. In another embodiment, the first cutting region and second cutting region may be connected in such a way that a centre of the second cutting region is positioned outboard of an imaginary line defining a perimeter of the first cutting region by distance that ranges from about 20% to about 40% of the second diameter. In these embodiments the first cutting region may have a diameter ranging from about 9 mm to about 15 mm and the second cutting region may have a diameter that ranges about 25% to about 45%, such as about 37.5%, of the diameter of the first cutting region. The straight walls may include regions that are curved, sinusoidal, circular or ovoid. In the embodiments the straight walls are replaced with curved, sinusoidal, circular or ovoid walls.

The first cutting region has a generally circular or ovoid shape. The second cutting region has a generally circular, ovoid or crescent shape. The punch may further comprise a third cutting region connected to the first cutting region. The second and third cutting regions may be positioned on opposite sides of the first cutting region. The third cutting region may have the same shape as the second cutting region. For example, if the second cutting region is generally circular, the third cutting region may also be generally circular. However, in some embodiments, the second and third cutting region may have different shapes. The second and third cutting region may be defined by respective structures made from the same material. In some embodiments the first, second and third cutting region may each have the same shape, such as circular or ovoid cross-sections.

The punch may further comprise a base connected to at least the structure of the first cutting region. In some embodiments the second and/or third cutting region may also be connected to the base. The base may further comprise a handle. The handle may be elongate. The base may have a flange above the blade to prevent inadvertent injury to tissues beneath the skin. For example, the flange may prevent the punch from being inserted beyond a maximum insertion depth.

Also disclosed herein is a therapeutic punch comprising: a first cutting region for forming a void in a piece of tissue; a second cutting region for cutting a flap associated with the void, the first and second cutting regions being defined by respective structures, the structure of the second cutting region being connected to the structure of the first cutting region; wherein the structure of the second cutting region is shaped and positioned relative to the structure of the first cutting region so that the flap is laterally transposable to cover the void.

Also disclosed herein is a therapeutic punch comprising: a first cutting region defined by a radially unsymmetrical cylinder having a first cutting edge for removing a section of tissue; a second cutting region extending from the first cutting region, the second cutting region being defined by a curved wall having a cutting edge for cutting a flap in a surface of the tissue next to the removed section of tissue, the curved wall of the second region having a curved profile that is similar to a curved profile of a portion of a wall of the first cutting region. The curved wall may also include a straight wall section extending therefrom.

A punch having the second cutting region for cutting a flap may provide cutting lines similar to that used in a classical transposition flap suturing technique. Transposition flap suturing is a technique that requires a skilled surgeon, and in some tissue types a skilled surgeon may still have trouble executing the correct cutting lines. The problem with transposition flap suturing is that it produces acutely angled corners in the skin which frequently necrose, and cause infection or poor healing. An embodiment of the punch may utilise the above principles and uses parts of a circle cutting (rounding) at the same time as the lesion is removed, so redundant excess tissue may be removed to produce the desirable "S" shaped cut and "S plasty". This may prevent the local complications which frequently occur with a transposition flap. An embodiment of the punch may be capable of making the correct cuts in a single application and may improve the quality of the cut and enable it to be performed by relatively untrained professionals with ease. Patient's cosmetic outcomes may be improved and the formation of wide/excess scar tissue and other disfiguring features may be reduced.

By "radially unsymmetrical" it is meant a cylinder having a cross-sectional area that is transverse to a longitudinal axis of the cylinder being defined by more than one radius. For example, radially unsymmetrical can include cross-sectional areas that resemble oval, elliptical, symmetric cam and teardrop shapes.

The first cutting region may be defined by a wall, such as a blade, with an upper edge forming the first cutting edge. The wall may have a first curved section defined by a first radius and a second curved section defined by a second radius. The first curved section and second curved section may be connected by wall segments. The wall segments may be straight and/or curved. The first radius may be larger than the second radius. The first cutting region may have a symmetric cam lobe cross-sectional shape. The second cutting region may be attached to the first cutting region near the first curved section. The second cutting region may be defined by a curved wall having an upper edge forming the second cutting edge. The curved wall may have a radius that is substantially similar to the first radius of the first curved section of the first cutting region. For example, the second cutting region may be a blade and/or cutter with a shape similar to a head of a symmetric cam lobe. The second cutting region may further comprise an auxiliary cutting region positioned at an apex of the curved wall of the second cutting region. The second cutting region may be connected to the first cutting region by a curved transition. The curved wall may have a straight wall portion that defines a terminus of the second cutting region.

The punch may further comprise a base to which the first and second cutting regions are attached. A handle may extend from the base. The handle may be elongate and may have a flange above the blade to prevent cutting of deep tissues. For example, the flange may act as a limit stop that defines a maximum insertion depth into tissue. The maximum insertion depth may be about 8 mm to about 10 mm.

In the embodiments of the disclosure, the first cutting region may have a diameter at its widest point ranging from about 6 mm to about 40mm. The first, second and/or cutting regions may be formed from any medical grade or otherwise material that can provide a cutting edge, such as metal(s), composite(s), plastic(s) and any combination thereof. The first and second cutting regions may be chamfered and/or have micro-serrations. This may help to improve the sharpness of the cutting regions to ensure a clean cut when a lesion is removed from tissue.

The disclosure also provides a package, such as a sterile package, comprising the punch as set forth above.

The disclosure also provides a method of manufacturing a therapeutic punch comprising: fixing a first cutting region to a base of the punch; and securing a second cutting region to the first cutting region.

The second cutting region may be secured to the first cutting region before fixing the first cutting region to the base. The second cutting region may be secured to the first cutting region by forming a lobe in the first cutting region to form the second cutting region. The method may further comprise fixing the second cutting region to the base. The method may further comprise securing a third cutting region to the first cutting region. The method may be used to form the therapeutic punch as set forth above.

### Brief description of Figures

Embodiments will now be described by way of example only with reference to the accompanying non-limiting Figures.
Figure 1a shows an embodiment of a therapeutic punch with flange and handle.
Figure 1b shows an end view of the punch from Figure 1a.
Figure 2 shows an embodiment of a therapeutic punch with flange and handle.
Figure 3a shows an embodiment of a therapeutic punch with flange and handle.
Figure 3b shows an embodiment of a cross-section profile of the therapeutic punch from Figure 3a.
Figure 3c shows an alternate embodiment of a cross-section profile of the therapeutic punch from Figure 3a.
Figure 3d shows an alternate embodiment of a cross-section profile of the therapeutic punch from Figure 3a.
Figure 3e shows an alternate embodiment of a cross-section profile of the therapeutic punch from Figure 3a.
Figure 4a shows an embodiment of a therapeutic punch with flange and handle.
Figure 4b shows a cross-section profile of the therapeutic punch from Figure 4a.
Figure 5a shows an embodiment of a therapeutic punch with flange and handle.
Figure 5b shows a cross-section profile of the therapeutic punch from Figure 5a.
Figure 6a shows an embodiment of a therapeutic punch.
Figure 6b shows a cross-section profile of the therapeutic punch from Figure 6a.
Figure 6c shows an embodiment of a cross-section profile of a therapeutic punch from Figure 6a.
Figure 7 shows a closure line in tissue resulting from the therapeutic punch from the cross-section of Figure 6c.

### Detailed description

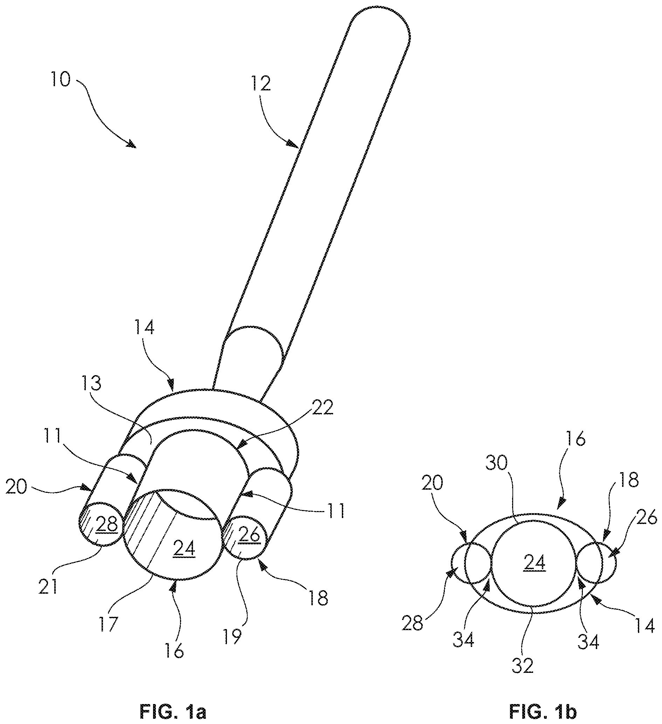

Figure 1 shows an embodiment of a therapeutic punch 10. The punch 10 has a handle in the form of elongate rod 12. The rod 12 tapers at one end to be joined to a base 14. The tapered region of rod 12 is not required in all embodiment. Extending from base 14 is a first cutting region in the form of main cutting tube 16. A first end 22 of the cutting tube 16 is fixed to the base 14, for example by embedding the tube 16 into the base 14, and/or through the use of fasteners and/or adhesives. Opposite to end 22 is a cutting edge 17 for cutting into tissue in use of the punch 10. A thickness of the cutting tube ranges from about 0.01mm to about 0.75 mm. The cutting edge 17 is chamfered and/or has micro-serrations in some embodiments. The main cutting tube 16 defines an internal volume 24. The cutting tube 16 is what a user would use to remove target tissue. The base 14 has a flange 13 that extends approximately transverse to a longitudinal direction of the handle 12. The flange 13 acts as a limit stop to prevent an incision/cut extending beyond a maximum depth. In such embodiments, this helps to prevent underlying deeper tissue from being damaged.

A second cutting region in the form of second cutting tube 18 is connected to an outer wall of the main cutting tube 16. The second cutting tube 18 defines an internal volume 26. The embodiment of Figure 1 also has a third cutting region in the form of third cutting tube 20 which is also connected to an outer wall of the main cutting tube 16. The third cutting tube 20 is positioned on the main cutting tube 16 to be approximately opposite, e.g. diametrically opposed, the position of the second cutting tube 18. The third cutting tube 20 defines an internal volume 28. The third cutting tube 20 may be not required in all embodiments.

An end view of the cutting tubes, as shown in Figure 1b, shows a cross-sectional area of the cutting tubes. The cutting tubes form a generally lissajous figure in cross-section. Diameters of each the second and third cutting tube 18/20 are less than a diameter of the main cutting tube 16. In some embodiments, a ratio of the diameter of the main cutting tube 16 to the diameter of the second cutting tube 18 and/or third cutting tube 20 ranges from about 1:0.25 to about 1:0.45, such as about 1:0.375. In use of punch 10, the main cutting tube 16 removes a section of tissue approximately similar to the internal volume 24. This in turn creates a main void in the tissue having the same dimensions as the removed section of tissue. At the same time, the second 18 and third 20 cutting tubes remove small sections of tissue on either side of the main void to form relief voids. The relief voids mean that when sides 30 and 32 of the main void (as best seen in Figure 1b) are sutured together, the tissue 34 proximate the intersect of the main void 24 can move to relieve any compressive and tensile forces exerted thereon. The effect of this is that puckering or dimpling of tissue surrounding the first cutting void formed by the punch 10 is reduced or even eliminated in some embodiments, and this can lead to improve patient outcomes due to the reduction of scar tissue. The cutting tube 16 has a diameter ranging from about 3 mm to about 15 mm.

The second and third cutting tubes have respective cutting edges 19 and 21. Similar to cutting edge 17, in some embodiments cutting edges 19 and 21 are chamfered and/or have micro-serrations in some embodiments. Cutting edges 17, 19 and 21 are all aligned on a common plane that is transverse to an axial direction of the main cutting tube 16. This arrangement means that the depth of each void formed by the main, second and third cutting tubes is the same, which helps to ensure that a depth of cut by the punch 10 is the same for each cutting tube. This helps to ensure the basal layer of the skin does not skew and is directly approximated. The common plane is spaced approximately 8 mm to 10mm from the flange 13. The thickest skin anywhere on the body is the upper back but it is never generally greater than 10mm, so a maximum insertion depth of about 10 mm can help to prevent inserting the punch into underlying tissue.

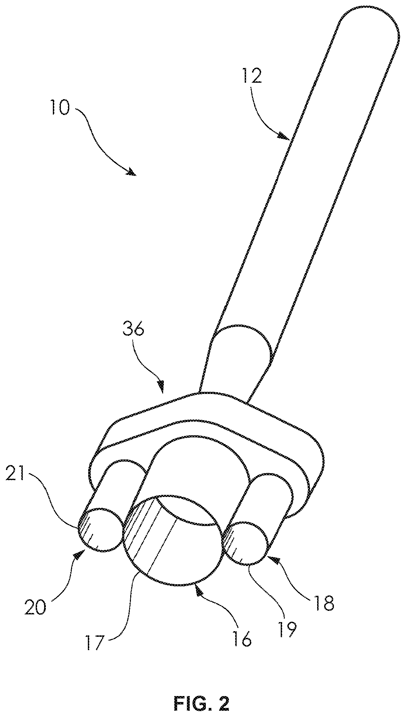

In the embodiment of Figure 1, the cutting tube 16 is embedded in the base 14 at the first end 22 and ends of the second 18 and third 20 cutting tube that are proximate the first end 22 are only partially embedded in the base, as best seen in Figure 1b. This allows the formation of open cutting tubes for the second and third cutting tubes 18/20. This allows the removal of all tissue as a single specimen. In other embodiments, only a portion of the first end 22 is embedded in the base 14. However, in other embodiments, such as that shown in Figure 2, the base 36 is sized so that each of the main 16, second 18 and third 20 cutting tubes are embedded in the base at the end opposite the cutting edges 17/19/21.

The main cutting tube 16 is generally cylindrical, however in other embodiments the main cutting tube are radially unsymmetrical e.g. having an oval or teardrop cross-sectional. In some embodiments, the second and third cutting tubes 18/20 have an oval or teardrop cross-section.

In the embodiments of Figures 1 and 2, the main cutting tube 16 and second and third cutting tubes 18/20 are formed from separate tubular sections and are connected to one another along connection line 11. The main cutting tube 16 is connected to the second 18 and third 20 cutting tubes along connection line 11 through the use of adhesives, such as medical-grade adhesives, welding and/or fasteners such as rivets, screws and bolts. To manufacture the punch 10, the second 18 and third 20 cutting tubes are secured to the main cutting tube 16 prior to fixing the main cutting tube 16 to the base 14. However, in some embodiments the second 18 and third 20 cutting tubes are secured to the main cutting tube 16 after the main cutting tube has been fixed to the base 14.

The main cutting tube 16 has a diameter at it widest point ranging from about 2 mm to about 25 mm. The second 18 and third 20 cutting tubes have a diameter ranging from about 0.5 mm to a maximum of 5mm. In some embodiments the diameter of the main cutting tube 16 determines the diameter of the second 18 and third 20 cutting tubes. For example, a ratio of the diameter of the main cutting tube to the diameter of the second 18 and/or third 20 cutting tubes may range from about 1:1.25 to about 1:1.45. As the diameter of the main cutting tube 16 increases, so too does the diameter of the second and third cutting tube in some embodiments.

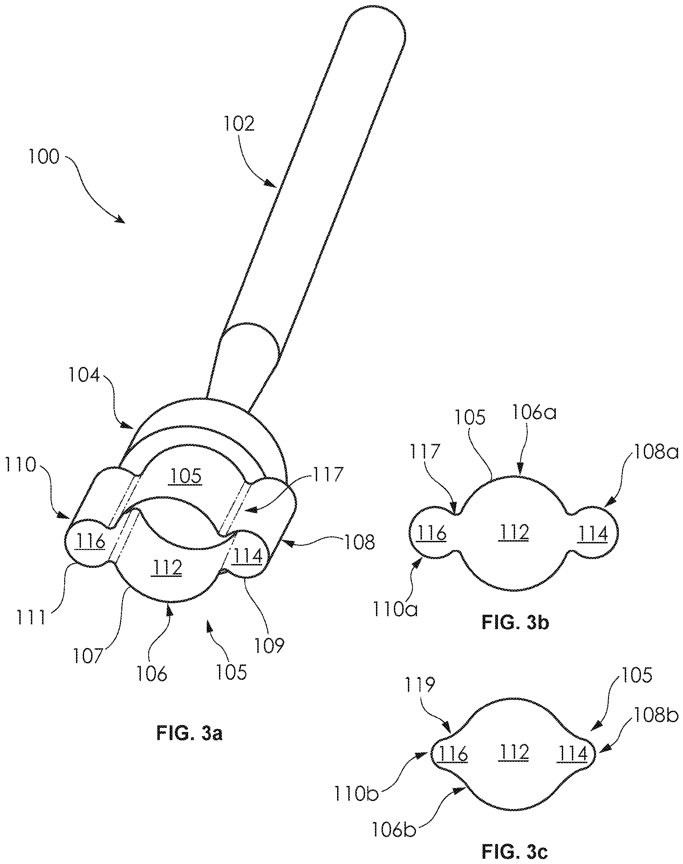

Figure 3a shows a different embodiment of a therapeutic punch. In this embodiment punch 100 has a first cutting region in the form of main cutting region 106 that is defined by a wall 105. On one side of the main cutting region 106 the wall 105 extends radially outwards to form a second cutting region in the form of lobe 108. The main cutting region 106 defines internal volume 112. The lobe 108 defines internal volume 114. Because there is no wall delineating internal volumes 112 and 114, as is the case for internal volumes 24 and 26 in the embodiment of Figure 1, the internal volume 114 of the lobe 108 extends into the internal volume112 of the main cutting region 106. In the embodiment of Figure 3a the wall 105 forms a single structure that extends as a continuous and unbroken wall to form the main cutting region 106 and lobe 108. A third cutting region in the form of lobe 110 is also formed by another radially extending region of the wall 105, so that an internal volume 116 of lobe 110 extends into the internal volume 112 of the main cutting region 106. The second lobe 108 and third lobe 110 are positioned on opposite sides of the main cutting tube 106.

Providing a single structure to form the main cutting region and lobes 108/110 can help to ensure a clean cut is performed in tissue when a sample of tissue is being taken using punch 100. A clean cut can help to ensure a cavity in tissue is formed having a straight, uniform and clean wall. A single wall structure may also help to simplify manufacture of the punch 100. A single structure may also help to meet the desiderata that a single piece of tissue is produced for the pathologist for histological examination.

A neck, or indent region 117, is formed at the junction between the main cutting region 106a and the lobes 108a and 110a, as seen in Figure 3b. However, in some embodiments no neck is formed between the main cutting region 106b and the lobes 108b and 110b, and instead a curved transition is formed by inflection point 119 between the main cutting region 106 and lobes 108 and 110, as seen in Figure 3c. In other embodiments (not shown), the indent region 117 forms a sharp transition e.g. a sharp point between the main cutting region 106 and the lobes 108 and/or 110.

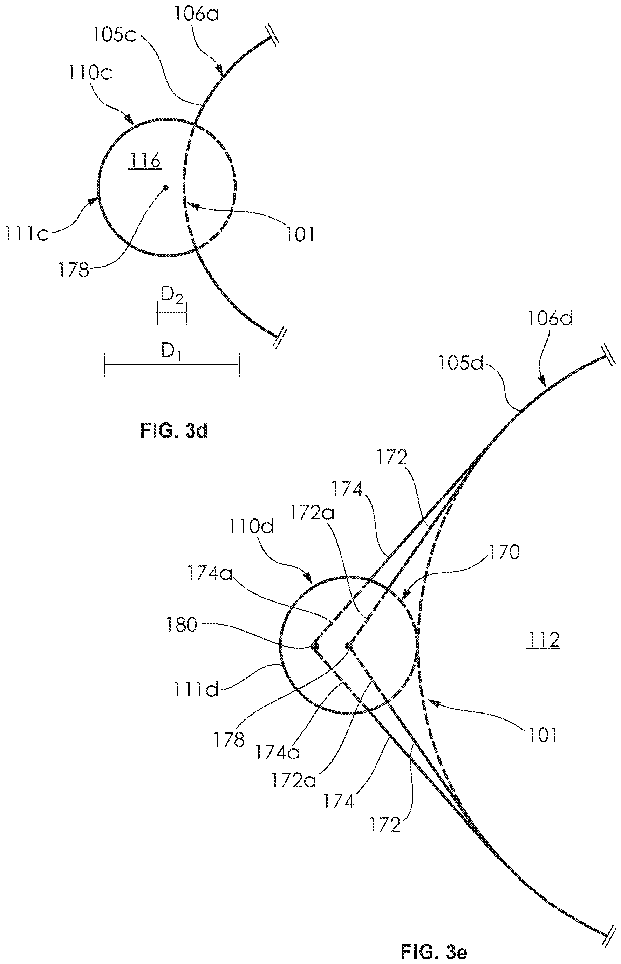

An alternative cross-section profile is shown in Figure 3d. Some features from the embodiment of Figure 3a have been omitted from Figure 3d for clarity, such as lobe 108. The main cutting region 106c has an approximately constant radius that is defined by the wall 105c. The wall 105c defines a perimeter of the main cutting region 106c. Where the lobe 110c extends from the main cutting region 106c, an imaginary perimeter of the main cutting region 106c is shown as dashed line 101. A centre of the lobe 110c is shown as point 178. The point 178 is positioned outboard of the perimeter 101 by a distance D₂ that is about 20% to about 40%, such as about 30%, of the diameter D₁ of the lobe 110c i.e. D₂ is approximately 0.3×D₁. In the embodiment of Figure 3d, the main cutting region 106c has a diameter ranging from 3 mm to 8 mm, where the lobe 110c has a diameter ranging from about 0.25 to about 0.45 times, such as 0.375 times, the diameter of the main cutting tube 106c.

Another alternative cross-section profile is shown in Figure 3e. In this embodiment, the lobe 110d is positioned so that a perimeter of the lobe 110d, as shown by dashed line 170, is touching the perimeter of the main cutting region 106d, as shown by dashed line 101. However, in some embodiments, dashed line 170 does not touch the perimeter of the main cutting region 106d, for example dashed line 170 may be spaced from main cutting region 106d. Alternatively, in some embodiments dashed line overlaps the perimeter of the main cutting region 106d. Straight wall sections 172 extends tangentially from the main cutting region 106d towards the lobe 110d. In one embodiment the straight wall sections 172 are positioned so that an imaginary line extending along each straight wall section, as shown by dashed line 172a intersects with a centre 178 of the lobe 110d. In another embodiment, straight wall sections 172 are replaced with straight wall sections 174. Straight wall sections 174 extend tangentially from main cutting region 106d and have imaginary lines 174a extending along each straight wall section that intersect at point 180. Not all embodiments require the straight wall sections 172 and 174 to extend tangentially from the main cutting region 106d. In some embodiments straight wall sections 172 and 174 are curved, circular and/or sinusoidal wall sections and/or are straight and comprise wall segments that are curved, circular and/or sinusoidal. Point 180 is positioned between centre 178 and a perimeter of the lobe 110d defined by the wall 111d. Embodiments of the cross-section profile of Figure 3e only have one set of walls per lobe (e.g. 172 or 174), but two sets of walls (172 and 174) are shown in Figure 3e for comparison purposes only. In most embodiments the intersect of the imaginary lines extending from the walls is positioned between the centre 178 and the wall 111d. In the embodiment of Figure 3e, the diameter of the main cutting region 106d ranges from approximately 9 mm to approximately 15 mm. In the embodiment of Figure 3e, lobe 110d has a diameter of up to a maximum of 5 mm.

The presence of the walls in Figure 3e provides an undercut that removes a cusp of tissue that allows tissue to "hinge" around the void left in the tissue by lobe 110d after a sample of tissue is removed from a patient. Put another way, the undercut region allows space for the tissue surrounding the undercut to fold into the region removed by the lobes. The wound length formed by the punch of Figures 3d and 3c are also reduced which helps to improve patient outcomes. This hinging ability means that the void formed by the punch 100 can be sutured together without worry of puckering, dimpling, or narcosis of tissue, which tends to occur in prior art punches in the region removed by lobe 110d.

It should be appreciated that although lobes 110c and 110d are shown as having a circular geometry in the embodiments of Figure 3d and Figure 3e, in some other embodiments lobes 110c and 110d have non-circular geometries including crescent and teardrop shapes.

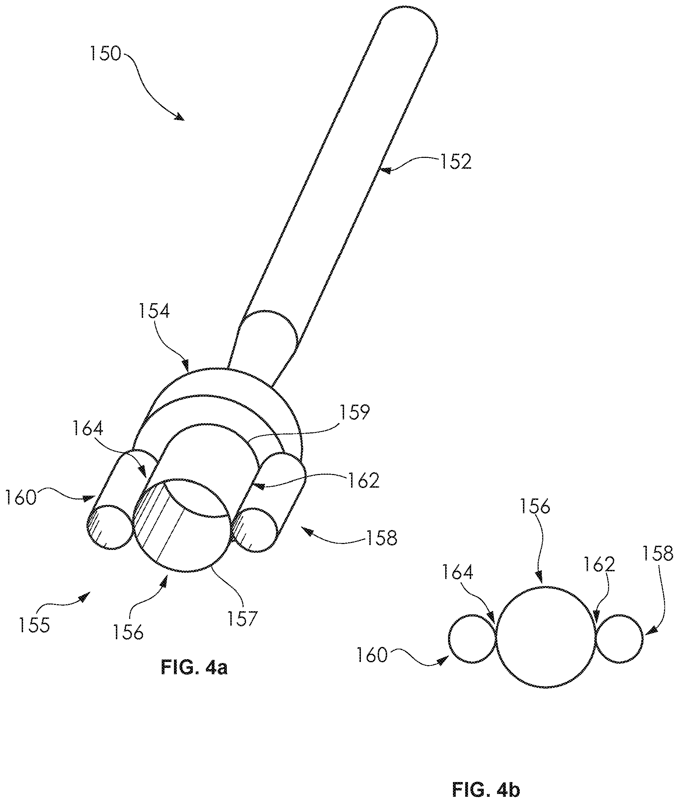

Figures 4a and 4b shows another embodiment of a punch. Punch 150 is similar to punch 10, but the cutting head 155 is formed from one continuous blade shaped to form a three-lobed cutter with a main cutting region 156 and diametrically opposed second cutting tube 158 and third cutting tube 160. The intersection of the lobes 158/160 with the main cutting region 156 is formed by slots extending parallel to an axial direction of the main cutting region 156 in one intersecting part from the cutting edge 157 to an edge 159 opposite the cutting edge, and in another intersecting part from the edge 159 to edge 157. The slots formed in the blade permit a cross over intersection to form intersects 162 which are formed between the main cutting region 156 and lobe 158 and intersect 164 formed between the main cutting region 156 and the lobe 160. The intersects 162 delimit the main cutting region 156 from the lobes 158/160. The lobes may help to de-bulk the tissue at the end of the wound that would otherwise form dog ears or dimples.

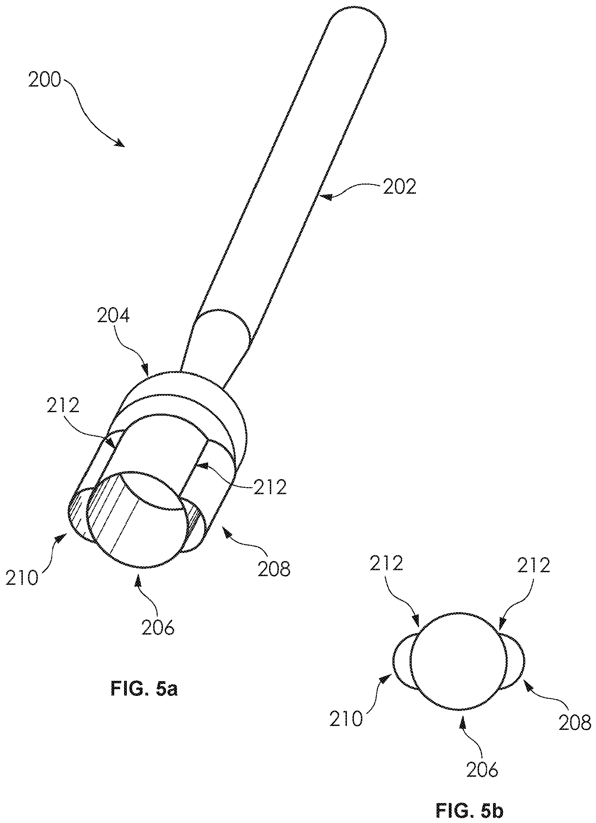

Figures 5a and 5b shows another embodiment of a punch. Punch 200 has a main cutting region in the form of main cutting tube 206 and a second and third cutting region in the form crescent cutting blades 208 and 210. Cutting blades 208 and 210 are positioned around the main cutting tube 206 diametrically opposed to one another. The crescent cutting blades 208 and 210 are partially embedded in base 204. Main cutting blade 206 is completely embedded in base 204. The main cutting blade 206 and crescent cutting blades 208 and 210 are welded together to form connection points 212. In some embodiments, adhesives and/or fasteners are used to connect the main cutting blade 206 and crescent cutting blades 208 and 210.

In the embodiments of Figures 1 to 5, the first and second cutting regions are depicted as having the same cross-sectional area and shape. In some alternative embodiments the first and second cutting regions have different cross-sectional areas. However, in these alternative embodiments, the first cutting region still has a larger diameter than the second and third cutting regions. Not all embodiments of the punches in Figures 1 to 5 have the third cutting region diametrically opposed the second cutting region. Some embodiments only have the second cutting region i.e. the third cutting region is omitted. In some embodiments the first, second and/or third cutting regions are non-circular, such as ovoid. In some embodiments each of the first, second and/or third cutting regions have different non-circular geometries.

The hole created by typical current punches are circular and therefore are not designed to close in a straight line. By using a second and optionally a third cutting tube in addition to the main cutting tube, it may be possible to form a circular void in tissue that can be closed in close to a straight line with minimal puckering and dimpling.

Compared with prior art punches, the embodiments of the punches described in Figures 1 to 5 may permit a much shorter incision length, for example at a (ratio of 1:1.6, much shorter than the usual 1:3-4) which may allow an accurate, clean, pucker and dimple free approximation of wound edges for diameters up to 25 mm with accurate approximation of the basal and dermal layers by sutures/glue along the length of the wound. Embodiments may allow a markedly reduced elastic tension forces to be correctly orientated to result in "fine line" good healing without the risk of delayed separation. There is generally no deformity of the skin, nor unsightly scaring, which can result with improved cosmetic results. Traditional surgical teaching dictates an elliptical excision (3-4 times the main diameter of the lesion plus margin), but the punches described herein provides a wound length of 1.6 times making a significant reduction in all aspects of wound management, healing and residual scarring. The convenience, speed, accuracy and operator confidence of one or more embodiments of the disclosed punch action is preserved yet cosmetic and clinical results are dramatically improved. With embodiments of the disclosure, time can be saved during lesion removal as well as a superior patient outcome.

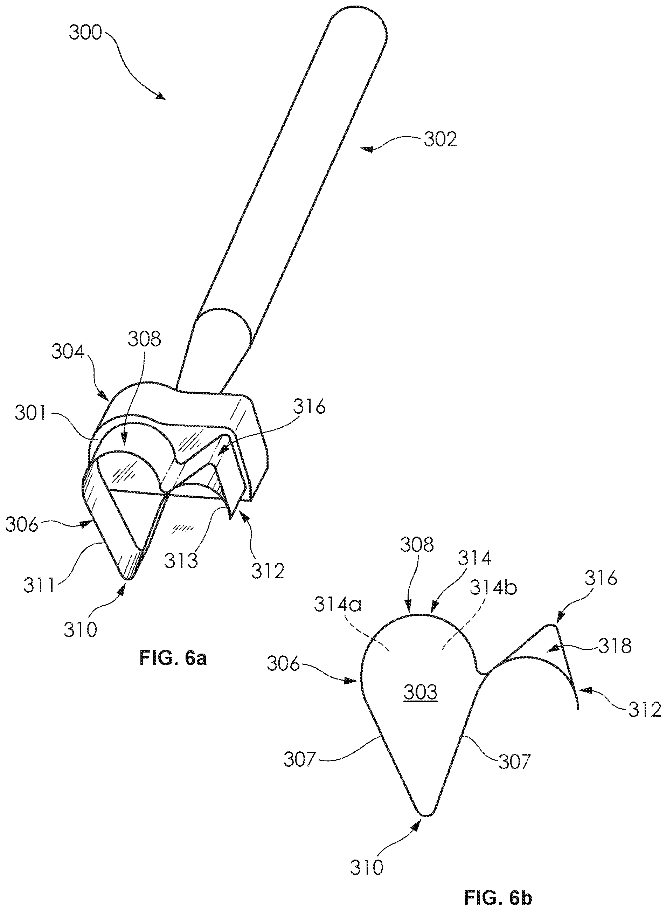

Another embodiment of a therapeutic punch is shown in Figure 6a and Figure 6b. Punch 300 has a handle 302 and base 304 similar to that of the embodiments described in Figures 1 to 5. The base defines a flange 301 that acts as a limit stop so that the punch 300 cannot be pressed into tissue so as to damage underlying tissue. The punch has a first cutting region in the form of symmetric cam lobe cutter 306. Symmetric cam lobe cutter 306 is a radially unsymmetrical cylinder that has a cutting edge 311 for removing a section of tissue, i.e. forming a void in a piece of tissue. The symmetric cam lobe cutter 306 is defined by a wall that has a first curved section in the form of bulbous end 308 that is defined by a first radius, and a second curved section in the form of pointed end 310 that is defined by a second radius. The first radius is larger than the second radius. The bulbous end 308 and the pointed end 310 are joined to one another by substantially straight wall segments 307 (see Figure 6b). In some embodiments straight wall segments 307 include curved regions or are alternative entirely curved. The symmetric cam lobe cutter 306 forms a symmetric cam lobe-shaped void in use of punch 300. The symmetric cam lobe cutter 306 is fixed to the base by an end opposite a cutting edge 311. The symmetric cam lobe cutter 306 has a diameter being that of the required extraction. For example, a diameter of the symmetric cam lobe cutter 306 ranges from about 6mm to about 35mm, with the diameter being any value within this range for example 10 mm, 11 mm, 12mm, 13mm and so on. In some embodiments, the diameter is larger than 35 mm. The cam lobe cutter 306 defines a void 303. Use of the punch 300 forms a void in tissue having similar dimensions and geometries to void 303.

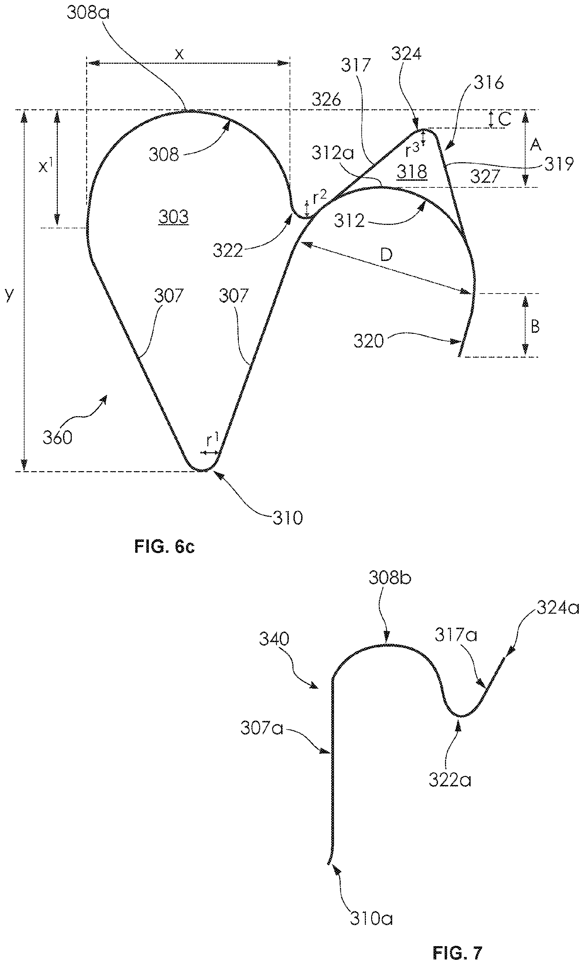

A second cutting region in the form of curved cutter 312 extends from the symmetric cam lobe cutter 306 at a region near the bulbous end 308. Curved cutter 312 is formed from a curved wall, where the curvature is defined approximately by a radius. In the embodiment of Figure 6, the curved cutter 312 is welded to the side wall of the symmetric cam lobe cutter 306. The curved cutter 312 is fixed to the base by an end opposite a cutting edge 313. The curved cutter 312 is for cutting a flap extending from an upper edge of the void of tissue removed by the symmetric cam lobe cutter 306. The cutting edge 313 of the curved cutter 312 is in the same plane as the cutting edge 311 of the symmetric cam lobe cutter 306 i.e. cutting edge 313 and 311 are aligned on a cutting plane. The cutting plane extends approximately transverse to an axial direction of the handle. The cutting plane is spaced approximately 8mm to 10mm from the flange 301.

In the embodiment of Figure 6a, the base 304 is connected to only a portion of the symmetric cam lobe cutter 306 so that the symmetric cam lobe cutter 306 is open ended. However, in other embodiments the symmetric cam lobe cutter 306 is fully connected i.e. embedded in base 304 so that it has a closed end. Generally, the curved cutter 316 is fully fixed in the base 304, but it some embodiments the curved cutter 316 is only partially fixed in the base 304.

The radius that approximately defines the curved cutter 312 is similar to curved profiled 314 that is defined between point 314a and 314b of the bulbous end 308. When a biopsy of tissue is removed in use of punch 300, a void is formed in the area removed by the symmetric cam lobe cutter 306 and a flap formed by curved cutter 312 sits next to an edge of the void. The shape of the curved cutter 312 permits a skin flap type closure rather than an unsightly graft with an accurate fitment and shape ensuring accurate clean, pucker, dimple, nodule free approximation of wound edges after sutures. The shape of the curved cutter 312 is of an appropriate size so that it forms a flap that if laterally transposed will fit within the major diameter of the symmetric cam lobe cutter 312.

At an apex of the curved cutter 312 there is an auxiliary cutting region in the form of secondary crescent cutter 316 that forms a void 318 extending from the apex of the curved cutter 312. The secondary crescent cutter 316 is for cutting a small void in the tissue that is positioned next to the flap of tissue formed by curved cutter 312. The small void formed by the secondary crescent cutter 316 removes excess tissue in one step, with the specimen and avoids the need for secondary adjustment with a scalpel to reduce puckering and dimpling when the voids formed punch 300 are sutured together.

Another embodiment of a cross-section profile of the punch 300 is shown in Figure 6c. Cam lobe cutter 306 had a principle diameter X and length Y. The length Y is approximately 1.5 to 2.0 times the diameter X. The bulbous end 308 is slightly oval in shape, where the diameter X is approximately 95% of the distance X'. Note that the Figure 6C is exemplary only and not drawn to scale. The pointed end 310 is defined by a curve having a radius r₁. The side walls 307 may be curved or straight. In the embodiment of Figure 6c, the side walls 307 are straight and extend tangentially from the pointed end 310, but the side walls 307 do not need to extend tangentially from pointed end 310 in all embodiments. The connection point between the bulbous end 308 and the curved cutter 312 is defined by transition curve 322. The transition curve 322 is defined by radius r₂. The curved cutter 312 has a diameter D that ranges from about 70% to about 100%, such as about 85%, of diameter X.

In the embodiment of Figure 6c, side wall 317 extends tangentially from transition curve 322. However, in some embodiments side wall 317 does not extend tangentially from transition curve 322. Side wall 317 can be curved or straight. Side wall 317 defines one side of the secondary crescent cutter 316, and side wall 319 defines a second side of the crescent cutter 316. The side walls 317 and 319 are connected to one another via apex 324. Apex 324 is defined by radius r₃. The space formed between the curved cutter 312, side walls 317 and 319, and apex 324 defines void 318. In the embodiment of Figure 6c, the apex 324 is positioned towards pointed end 310 with respect to plane 326 by distance C (plane 324 is the plane of apex 308a of the bulbous end 308). Distance C is about 10% to about 15% of distance Y. An apex 312a of the curved cutter 312 is located on a plane 327 that is positioned towards the pointed end 310 with respect to the apex 308a. The apex 312a is spaced from the apex 308a of the bulbous part 308 in the Y direction by distance A. Distance A ranges from about 10% to about 20%, such as 15%, of distance Y. In the embodiment of Figure 6c, the curved cutter 312 terminates with a straight wall segment 320. Wall segment 320 extends in the Y direction by length B. Length B ranges from about 10% to about 35%, such as about 25%, of distance Y. A distance that starts at apex 308a and extends along a perimeter of the wall of the bulbous end 308 and terminates at transition curve 322 is approximately the same as a distance extending along a wall portion from apex 324 to a terminus of wall segment 320.

The pointed end 310, transition curve 322, apex 324 each have a diameter defined by twice their radius e.g. two times r₁, r₂ and r₃, respectively. The diameters range from about 25% to about 45%, such as approximately 37.5%, the diameter X, up to a maximum of 5.0 mm.

The cam lobe cutter 306, curved cutter 312 and secondary crescent cutter 316 in some embodiments are formed from a single section of material, such as stainless steel having a thickness of about 0.01mm to about 0.75 mm, that is bent to form each of the features of the punch 300.

The following refers to voids and cut lines formed in a tissue in use of punch 300. Reference numerals used to describe features of the punch 300 will now be used to refer to respective features formed in tissue by punch 300. Use of the punch shown in Figure 6c forms a void in the tissue defined by void 303. In these embodiments, the tissue flap that is formed by the cam lobe cutter 312 can be further cut e.g. with a scalpel to separate the skin from the underlying tissue, but this is not required in all embodiments. Another void in the tissue is also defined by void 318. Voids 303 and 318 are formed once the tissue has been cut with the punch 300 and the tissue in regions 303 and 318 are discarded i.e. removed from the tissues. In the case of a therapeutic procedure, the discarded tissue is submitted for pathology examination. Once the voids 303 and 318 have been formed in the tissue, and optionally the skin is cut away from the underlying tissue in the tissue flap e.g. side walls 307, the sides of the voids are sutured together to close the void 303 so that apex 312a is sutured in a position that is located proximal to apex 308a. At the same time, a side extending from apex 324 along side wall 319 down to terminus 320 is sutured to side wall 317 to close void 318. The resulting single, unbroken, cut line 340 resembles an "S" shape, as shown in Figure 7. Figure 7 shows how the features of the punch 300 result in the S shape. For example, the side walls 307 when sutured together form cut line portion 307a. Suturing the apex 312a near apex 308a forms curved cut line portion 308b, and suturing the side extending from apex 324 along side wall 319 down to terminus 321 to side wall 317 forms cut line portion 317a. A second curved cut line portion 322a is formed by transition curve 322 into terminus 320. The single, unbroken, cut line has terminus 310a that is formed by pointed end 310 and terminus 324a formed by apex 324.

Because the curved cutter 312 that has a diameter D that is approximately 85% of diameter X, when the flap cut by curved cutter 312 is sutured to the form cut segments 307a and 308a, the smaller size of the flap with respect to void 303 provides a tension that pulls the tissue together. If the diameter D was the same as diameter X relaxation of the tissue would generally result in dimples and other such unfavourable features, resulting in a poor cosmetic result.

The punch shown in Figure 6c removed excess tissue at transition 322 and void 318 in one process which obviates the need for addition cutting with a scalpel normally undertaken to prevent "dog ears" found with existing translation flap procedures. Further, the smooth curved nature if all the intersections points of punch 300 prevents skin necrosis commonly occurring with sharp corners and distributes the skin stresses resulting in a flat fine line scar in some embodiments. This helps to provide a good cosmetic result.

The shape of the cutters 306 and 312 forms the elements classically used with translation flap procedures. But also eliminates acute angles and tissue necrosis. In some embodiments punch 300 is suitable for extractions of tissue up to 35+ mm in principal diameter, and extractions of tissue in excess of 10mm to 15mm on any anatomical site as well as 6mm+ lesions on the face, or the leg and foot. Further, the cutting shape created by punch 300 cannot be created using a scalpel blade as smooth curves transitions cannot be made with a scalpel. The wound shape permits a skin flap type closure rather than an unsightly graft with an accurate fitment and shape ensuring accurate clean, pucker, dimple, nodule free approximation of wound edges after sutures. The cutters 306 and 312 avoid the dangers of ischaemic tissue in cut acute angles that occur when a scalpel is used. Instead punch 300 provides smooth rounded small diameter transitions and extracts skin that would otherwise be subject to necrosis and remove excess tissue which normally has to be cut out at the ends as it is sutured. The resulting "S" shape reduces skin stresses and hypertrophic scarring. It produces "fine line" good healing without tension and risk of delayed separation. In some embodiments use of the punch 300 results in no deformity of the skin to cause unsightly scaring, which provides improved cosmetic results. Curved scars are visually less conspicuous than straight line scars and therefore preferred. This improved shape of the wound cannot be achieved accurately with a scalpel or a circular punch. The convenience, speed, accuracy and operator confidence of a punch action is preserved yet cosmetic and clinical results are excellent.

The embodiments of Figures 6a to 6c all show the curved cutter 312 extending from a right-hand side of the cam lobe cutter 306. However, in some embodiments the curved cutter 312 extends from a left-hand side of the cam lobe cutter 306. The resulting "S" cut line from the "left-hand" punch would be a mirror image to the cut line shown in Figure 7. The tissue type may determine whether a left- or right-hand punch is required.

The various punches described herein are all "single use only", i.e. disposable. Such punches are generally provided in a sterile package. In these embodiments the handle is generally formed from a medial grade plastic, and the various cutting edges and blades are formed from stainless steel. The disposable punches are generally provided in a sterile package that comprises the punch. However, in some embodiments the punches described herein may not be disposable, but this is generally not recommended due potential cross infection. In these embodiments, the punches are made from a material, such as stainless steel, carbon fibre or other that can be sterilised by typical techniques such as gas sterilisation, liquid sterilisation and autoclaving.

It will be understood to persons skilled in the art of the disclosure that many modifications may be made without departing from the scope of the disclosure.

In the claims which follow and in the preceding description, except where the context requires otherwise due to express language or necessary implication, the word "comprise" or variations such as "comprises" or "comprising" is used in an inclusive sense, i.e. to specify the presence of the stated features but not to preclude the presence or addition of further features in various embodiments.

## Claims

1. A therapeutic punch (10,100,150,200,300) comprising:
a first structure (16,106,156,206,306) defining a first cutting region which is generally circular or ovoid and is configured in use to remove a first section of tissue and a second structure (18,108,158,208,312) defining a second cutting region which is generally circular, ovoid or crescent-shaped and is configured in use to remove a second section of tissue, the first cutting region and second cutting region being separate from and connected to one another, **characterised in that**
the second cutting region has a diameter that is smaller than the first cutting region and that the second cutting region comprises a lobe (108, 108a, 108b,158,208,312) extending from the first cutting region and the shape of the cutting regions being configured such that closure of the tissue in the second section of tissue reduces puckering, rippling or dimpling of tissue surrounding the first section of tissue.

2. The punch of claim 1, wherein the first cutting region has a diameter ranging from about 3 mm to about 8 mm and the second cutting region has a diameter that ranges from about 25% to about 45% of the diameter of the first cutting region.

3. The punch of claim 1 or 2, wherein the first cutting region and second cutting region are connected in such a way that a centre of the second cutting region is positioned outboard of an imaginary line defining a perimeter of the first cutting region by distance that is about 20% to about 40% of the second diameter.

4. The punch of claim 3, wherein the first cutting region has a diameter ranging from about 9 mm to about 15 mm and the second cutting region has a diameter that is about 25% to about 45% of the diameter of the first cutting region.

5. The punch of any one of claims 1 to 4, further comprising a third cutting region (20,110,110,a,110b,160,210) connected to the first cutting region.

6. The punch of claim 5, wherein the second and third cutting regions are positioned on opposite sides of the first cutting region.

7. The punch of claim 5 or 6, wherein the third cutting region has a same shape as the second cutting region and is made from the same material as the second cutting region.

8. A method of manufacturing a biopsy punch (10,100,150,200,300) as defined in claim 1 comprising:
fixing to a base (14,36,104,154,204,304) of the punch a first structure defining a first cutting region which is generally circular or ovoid and is configured in use to remove a first section of tissue; and
connecting a second structure to the first structure before fixing the first structure to the base or to the base of the punch, the second structure defining a second cutting region which is generally circular, ovoid or crescent-shaped and configured in use to remove a second section of tissue,
such that the first cutting region and second cutting region are separate from and connected to one another, wherein the second cutting region is configured such it comprises a lobe extending from the first cutting region.

9. The method of claim 8, wherein the second structure is secured to the first structure before fixing the first structure to the base.

10. The method of claim 8 or 9, wherein the second structure is secured to the first cutting region by forming a lobe in the first structure to form the second structure.

11. The method of any one of claims 8 to 10 further comprising fixing the second structure to the base.

12. The method of any one of claims 8 to 11, further comprising securing a third structure (20,110,110,a,110b,160,210) to the first structure.

13. A biopsy punch (10,100,150,200,300) formed by the method of any one of claims 8 to 12.

## Patentansprüche

1. Therapeutische Stanze (10, 100, 150, 200, 300), die Folgendes umfasst:
eine erste Struktur (16, 106, 156, 206, 306), die einen ersten Schneidbereich definiert, der im Wesentlichen kreisförmig oder oval ist und dazu konfiguriert ist, einen ersten Gewebeabschnitt zu entfernen, und eine zweite Struktur (18, 108, 158, 208, 312), die einen zweiten Schneidbereich definiert, der im Wesentlichen kreisförmig, oval oder halbmondförmig ist und dazu konfiguriert ist, einen zweiten Gewebeabschnitt zu entfernen, wobei der erste Schneidbereich und der zweite Schneidbereich voneinander getrennt und miteinander verbunden sind, **dadurch gekennzeichnet, dass** der zweite Schneidbereich einen Durchmesser aufweist, der kleiner als der erste Schneidbereich ist, und dadurch, dass der zweite Schneidbereich eine Ausbuchtung (108, 108a, 108b, 158, 208, 312) umfasst, die sich vom ersten Schneidbereich erstreckt, und die Form der Schneidbereiche derart konfiguriert ist, dass der Verschluss des Gewebes im zweiten Gewebeabschnitt eine Faltenbildung, Wellenbildung oder Dellenbildung im Gewebe, das den ersten Gewebeabschnitt umgibt, reduziert.

2. Stanze nach Anspruch 1, wobei der erste Schneidbereich einen Durchmesser aufweist, der ungefähr 3 mm bis ungefähr 8 mm beträgt, und der zweite Schneidbereich einen Durchmesser aufweist, der ungefähr 25% bis ungefähr 45% des Durchmessers des ersten Schneidbereichs beträgt.

3. Stanze nach Anspruch 1 oder 2, wobei der erste Schneidbereich und der zweite Schneidbereich derart miteinander verbunden sind, dass die Mitte des zweiten Schneidbereichs außerhalb einer imaginären Linie positioniert ist, die den Umfang des ersten Schneidbereichs durch einen Abstand definiert, der ungefähr 20% bis ungefähr 40% des zweiten Durchmessers beträgt.

4. Stanze nach Anspruch 3, wobei der erste Schneidbereich einen Durchmesser aufweist, der ungefähr 9 mm bis ungefähr 15 mm beträgt, und der zweite Schneidbereich einen Durchmesser aufweist, der ungefähr 25% bis ungefähr 45% des Durchmessers des ersten Schneidbereichs beträgt.

5. Stanze nach einem der Ansprüche 1 bis 4, ferner umfassend einen dritten Schneidbereich (20, 110, 110a, 110b, 160, 210), der mit dem ersten Schneidbereich verbunden ist.

6. Stanze nach Anspruch 5, wobei der zweite und der dritte Schneidbereich auf gegenüberliegenden Seiten des ersten Schneidbereichs positioniert sind.

7. Stanze nach Anspruch 5 oder 6, wobei der dritte Schneidbereich die gleiche Form wie der zweite Schneidbereich aufweist und aus dem gleichen Material wie der zweite Schneidbereich hergestellt ist.

8. Verfahren zum Herstellen einer wie in Anspruch 1 definierten Biopsiestanze (10, 100, 150, 200, 300), das Folgendes umfasst:
Befestigen einer ersten Struktur, die einen ersten Schneidbereich definiert, der im Wesentlichen kreisförmig oder oval ist und dazu konfiguriert ist, einen ersten Gewebeabschnitt zu entfernen, an einer Basis (14, 36, 104, 154, 204, 304) der Stanze; und
Verbinden einer zweiten Struktur mit der ersten Struktur, bevor die erste Struktur an der Basis befestigt wird, oder mit der Basis der Stanze, wobei die zweite Struktur einen zweiten Schneidbereich definiert, der im Wesentlichen kreisförmig, oval oder halbmondförmig ist und dazu konfiguriert ist, einen zweiten Gewebeabschnitt zu entfernen,
so dass der erste Schneidbereich und der zweite Schneidbereich voneinander getrennt und miteinander verbunden sind, wobei der zweite Schneidbereich derart konfiguriert ist, dass er eine Ausbuchtung umfasst, die sich vom ersten Schneidbereich erstreckt.

9. Verfahren nach Anspruch 8, wobei die zweite Struktur an der ersten Struktur gesichert wird, bevor die erste Struktur an der Basis befestigt wird.

10. Verfahren nach Anspruch 8 oder 9, wobei die zweite Struktur an dem ersten Schneidbereich gesichert wird, indem eine Ausbuchtung in der ersten Struktur gebildet wird, um die zweite Struktur zu bilden.

11. Verfahren nach einem der Ansprüche 8 bis 10, ferner umfassend das Befestigen der zweiten Struktur an der Basis.

12. Verfahren nach einem der Ansprüche 8 bis 11, ferner umfassend das Sichern einer dritten Struktur (20, 110, 110a, 110b, 160, 210) an der ersten Struktur.

13. Biopsiestanze (10, 100, 150, 200, 300), die durch das Verfahren nach einem der Ansprüche 8 bis 12 gebildet wird.

## Revendications

1. Poinçon thérapeutique (10, 100, 150, 200, 300) comprenant :
une première structure (16, 106, 156, 206, 306) définissant une première région de coupe qui est généralement circulaire ou ovoïde et est configurée en utilisation pour retirer une première section de tissu et une deuxième structure (18, 108, 158, 208, 312) définissant une deuxième région de coupe qui est généralement circulaire, ovoïde ou en forme de croissant et est configurée en utilisation pour retirer une deuxième section de tissu, la première région de coupe et la deuxième région de coupe étant séparées et reliées l'une à l'autre, **caractérisé en ce que**
la deuxième région de coupe présente un diamètre qui est inférieur à celui de la première région de coupe et **en ce que** la deuxième région de coupe comprend un lobe (108, 108a, 108b, 158, 208, 312) s'étendant depuis la première région de coupe et la forme des régions de coupe étant configurée de sorte que la fermeture du tissu dans la deuxième section de tissu réduise le plissement, l'ondulation ou le capitonnage du tissu entourant la première section de tissu.

2. Poinçon selon la revendication 1, dans lequel la première région de coupe présente un diamètre variant d'environ 3 mm à environ 8 mm et la deuxième région de coupe présente un diamètre qui varie d'environ 25% à environ 45% du diamètre de la première région de coupe.

3. Poinçon selon la revendication 1 ou 2, dans lequel la première région de coupe et la deuxième région de coupe sont reliées de telle manière qu'un centre de la deuxième région de coupe soit positionné à l'extérieur d'une ligne imaginaire définissant un périmètre de la première région de coupe d'une distance qui est d'environ 20% à environ 40% du deuxième diamètre.

4. Poinçon selon la revendication 3, dans lequel la première région de coupe présente un diamètre variant d'environ 9 mm à environ 15 mm et la deuxième région de coupe présente un diamètre qui est d'environ 25% à environ 45% du diamètre de la première région de coupe.

5. Poinçon selon l'une quelconque des revendications 1 à 4, comprenant en outre une troisième région de coupe (20, 110, 110a, 110b, 160, 210) reliée à la première région de coupe.

6. Poinçon selon la revendication 5, dans lequel les deuxième et troisième régions de coupe sont positionnées sur des côtés opposés de la première région de coupe.

7. Poinçon selon la revendication 5 ou 6, dans lequel la troisième région de coupe présente une forme identique à celle de la deuxième région de coupe et est réalisée en le même matériau que la deuxième région de coupe.

8. Procédé de fabrication d'un poinçon de biopsie (10, 100, 150, 200, 300) selon la revendication 1, comprenant :
la fixation à une base (14, 36, 104, 154, 204, 304) du poinçon d'une première structure définissant une première région de coupe qui est généralement circulaire ou ovoïde et est configurée en utilisation pour retirer une première section de tissu ; et
la connexion d'une deuxième structure à la première structure avant la fixation de la première structure à la base ou à la base du poinçon, la deuxième structure définissant une deuxième région de coupe qui est généralement circulaire, ovoïde ou en forme de croissant et configurée en utilisation pour retirer une deuxième section de tissu,
de sorte que la première région de coupe et la deuxième région de coupe soient séparées l'une de l'autre et reliées l'une à l'autre, dans lequel la deuxième région de coupe est configurée de sorte qu'elle comprenne un lobe s'étendant depuis la première région de coupe.

9. Procédé selon la revendication 8, dans lequel la deuxième structure est fixée à la première structure avant la fixation de la première structure à la base.

10. Procédé selon la revendication 8 ou 9, dans lequel la deuxième structure est fixée à la première région de coupe par formation d'un lobe dans la première structure pour former la deuxième structure.

11. Procédé selon l'une quelconque des revendications 8 à 10, comprenant en outre la fixation de la deuxième structure à la base.

12. Procédé selon l'une quelconque des revendications 8 à 11, comprenant en outre la fixation d'une troisième structure (20, 110, 110a, 110b, 160, 210) à la première structure.

13. Poinçon de biopsie (10, 100, 150, 200, 300) formé par le procédé selon l'une quelconque des revendications 8 à 12.
